(19) Europäisches Patentamt
European Patent Office
Office européen des brevets

(11) Veröffentlichungsnummer: **0 396 134 A1**

(12) # EUROPÄISCHE PATENTANMELDUNG

(21) Anmeldenummer: **90108372.5**

(51) Int. Cl.[5]: **C07D 223/22**

(22) Anmeldetag: **04.05.90**

(30) Priorität: **04.05.89 HU 215889**

(43) Veröffentlichungstag der Anmeldung: **07.11.90 Patentblatt 90/45**

(84) Benannte Vertragsstaaten: **CH DE ES FR GB IT LI**

(71) Anmelder: **ALKALOIDA VEGYESZETI GYAR**
**Kabay János ut 27-29**
**H-4440 Tiszavasvári(HU)**

(72) Erfinder: **Cziáky, Zoltán,**
**Kelp I. u. 1/B, 1/4,**
**H-4440 Tiszavasvári,(HU)**
Erfinder: **Horváth, András,**
**Elmunkás, u. 7,**
**H-4440 Tiszavasvári,(HU)**
Erfinder: **Korodi, Ferenc,**
**Elmunkás u. 9, II/3,**
**H-4440 Tiszavasvári,(HU)**
Erfinder: **Kohegyi, Imre,**
**Elmunkás u. 11, II/4,**
**H-4440 Tiszavasvári,(HU)**
Erfinder: **Szabo, Zoltán,**
**Kishegyesi u. 68 fsz. 2,**
**H-4029 Debrecen,(HU)**
Erfinder: **Timár, Tibor Dr.,**
**Kabay J. u. 17-19, 1/6,**
**H-4440 Tiszavasvári,(HU)**
Erfinder: **Frank, Lászlo Dr.,**
**Kabay J. u. 17-19, III/10,**
**H-4440 Tiszavasvári,(HU)**
Erfinder: **Galamb, Vilmos Dr.,**
**Kelp I. u. 3/c,**
**H-4440 Tiszavasvári,(HU)**
Erfinder: **Balogh-Korik, Piroska,**
**Csokonai u. 1,**
**H-4450 Tiszalök,(HU)**
Erfinder: **Fekete, Miklos,**
**Elmunkás u. 1, II/B,**
**H-4440 Tiszavasvári,(HU)**
Erfinder: **Horváth, Sándor,**
**Aradi Vértanuk u. 35,**
**H-4440 Tiszavasvári,(HU)**
Erfinder: **Juhász, Károly,**
**Vizmü u. 14,**
**H-4440 Tiszavasvári,(HU)**
Erfinder: **Tamás, Lajos,**
**Dozsa Gy. u. 49,**
**H-4440 Tiszavasvári,(HU)**
Erfinder: **Terebes, Lászlo,**
**Kinizsi u. 6/b,**
**H-4440 Tiszavasvári,(HU)**

(74) Vertreter: **Beszédes, Stephan G., Dr.**
**Patentanwalt, Münchener Strasse 80a**
**D-8060 Dachau(DE)**

(54) **Verfahren zur Herstellung von 5H-Dibenz (b,f) azepin.**

(57) Gegenstand der Erfindung ist ein Verfahren zur Berstellung von 5H-Dibenz[b,f]azepin der Formel

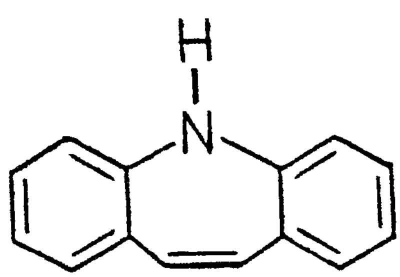

I

EP 0 396 134 A1

durch Dehydrieren von 10,11-Di-(hydro)-5H-dibenz[b,f]azepin der Formel

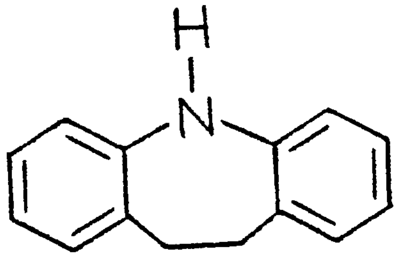

II

in Gegenwart von 1 oder mehr Edelmetallkatalysator(en) und Wasserstoffakzeptor(en) und gegebenenfalls Lösungsmittel(n), bei welchem die Umsetzung in der Schmelze oder in 1 oder mehr hochsiedenden polaren protischen Lösungsmittel(n) oder einer Mischung aus 1 oder mehr hochsiedenden polaren protischen Lösungsmittel(n) und 1 oder mehr apolaren Lösungsmittel(n) mit höherem beziehungsweise höheren Siedepunkt(en) als der beziehungsweise die des beziehungsweise der ersteren bei Temperaturen nicht über 300°C und bei Verwendung des Wasserstoffakzeptors beziehungsweise der Wasserstoffakzeptoren in Mengen von 0,35 bis 2 Mol je Mol des 10,11-Di-(hydro)-5H-dibenz[b,f]azepines der Formel II durchgeführt wird.

## Verfahren zur Herstellung von 5H-Dibenz[b,f]azepin

Die Erfindung betrifft ein besseres Verfahren zur Herstellung von 5H-Dibenz[b,f]azepin der Formel

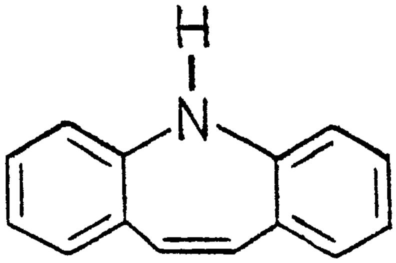

I ,

allgemein bekannt unter der Bezeichnung Iminostilben, durch in flüssiger Phase vorgenommenes katalytisches Dehydrieren von 10,11-Di-(hydro)-5H-dibenz[b,f]azepin (Iminodibenzyl) der Formel

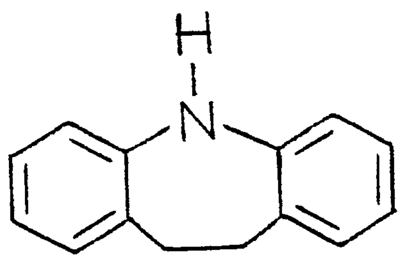

II .

Es sind bereits auf katalytischem Dehydrieren beruhende Verfahren zum in einem Schritt erfolgenden Umsetzen von 10,11-Di-(hydro)-5H-dibenz[b,f]azepin zu 5H-Dibenz[b,f]azepin bekannt.

So wird gemäß den US-PS 3 074 931 und 3 449 324, DE-PS 1 545 736 und JA-PS 80 17 330 das katalytische De hydrieren in der Gasphase in Gegenwart von Metallen oder Metalloxyden als Katalysatoren bei 350 bis 600°C vorgenommen; die Umsetzung ist jedoch unvollständig.

Gemäß der EP-OS 237 952 wird das katalytische Dehydrieren von 10,11-Di-(hydro)-5H-dibenz[b,f]-azepin zu 5H-Di-benz[b,f]azepin in der flüssigen Phase vorgenommen. Als Katalysatoren werden Edelmetalle oder -metalloxyde, als Lösungsmittel Diphenyläther oder -metalloxyde, als Wasserstoffakzeptoren Nitrotoluole, Diäthylmaleat oder Tetrachlorbenzochinon verwendet. Gemäß der Beschreibung verläuft die Reaktion bei 210 bis 250°C in 3 bis 5 Stunden. Eigene Reproduktionsversuche der angegebenen Beispiele zeigten indessen, daß unter den angegebemem Re aktionsbedingungen die Umsetzung höchstens 60 bis 65%-ig is und auch durch wesentlich längere Reaktionszeiten nicht erhöht werden kann. Wie die Reproduktion zeigte, kommt es bei einer 2 Stunden übersteigenden Reaktionszeit zu einer bedeutenden Zersetzung, welche die Ausbeute schmälert und die Qualität des produktes stark verschlechtert. Im technischen beziehungsweise industriellen Maßstab kann auf diese Weise das Produkt nur in schwacher Ausbeute und schlechter Qualität hergestellt werden. Die während der langen Reaktionszeit auftretende Zersetzung, der große Überschuß des verwendeten Wasserstoffakzeptors und das ungeeignet gewählte Lösungsmittel vergiften den Katalysator, der deshalb nicht wiederverwendet werden kann. Zusammenfassend ist festzustellen, daß dieses Verfahren zur wirtschaftlichen Herstellung von 5H-Dibenz[b,f]azepin in technischem beziehungsweise industriellem Maßstabe nicht geeignet ist.

Der Erfindung liegt daher die Aufgabe zugrunde, ein besseres und wirtschaftlicheres Verfahren zur Herstellung von 5H-Dibenz[b,f]azepin durch katalytisches Dehydrieren von 10,11-Di-(hydro)-5H-dibenz[b,f]-azepin, durch welches das erstere mit höherer Umsetzung und damit in höherer Ausbeute und Reinheit ohne oder nur mit eingeschränkter Vergiftung des Katalysators bei geringerer Reaktionsdauer erhalten werden kann und welches auch im Betriebsmaßstab durchgeführt werden kann zu schaffen.

Das Obige wurde überraschenderweise durch die Erfindung erreicht.

Die Erfindung beruht auf der überraschenden Feststellung, daß die Zersetzung des Produktes von der Menge des Wasserstoffakzeptors stärker beeinflußt wird als von der Erhöhung der Temperatur. Es konnte ferner überraschenderweise festgestellt werden, daß keine Zersetzung des Produktes eintritt, wenn die

Umsetzung in der Schmelze oder in einer flüssigen Phase besonderer Zusammensetzung bei Temperaturen nicht über 300°C und in Gegenwart einer verringerten Wasserstoffakzeptormenge vorgenommen wird.

Ferner beruht die Erfindung auf der überraschenden Feststellung, daß beim Arbeiten in der Schmelze oder in einem hochsiedenden Lösungsmittel die Menge des Wasserstoffakzeptors verringert werden kann, die Vergiftung des Katalysators vermeidbar oder einschränkbar ist, die Umsetzung beträchtlich erhöht und die Reaktionsdauer kürzer ist und das Produkt in guter Ausbeute und ausgezeichneter Qualität erhalten werden kann.

Gegenstand der Erfindung ist daher ein Verfahren zur Herstellung von 5 H-Dibenz[b,f]azepin der Formel

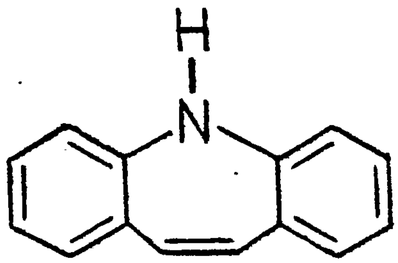

I

durch Dehydrieren von 10,11-Di-(hydro)-5H-dibenz[b,f]azepin der Formel

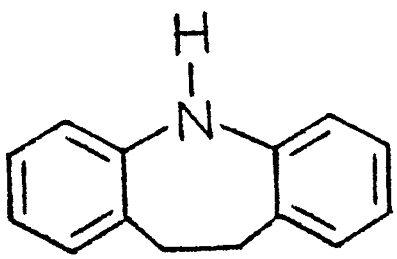

II

in Gegenwart von 1 oder mehr Edelmetallkatalysator(en) und Wasserstoffakzeptor(en) und gegebenenfalls Lösungsmittel(n), welches dadurch gekennzeichnet ist, daß die Umsetzung in der Schmelze oder in 1 oder mehr hochsiedenden polaren protischen Lösungsmittel(n) oder einer Mischung aus 1 oder mehr hochsiedenden polaren protischen Lösungsmittel(n) und 1 oder mehr apolaren Lösungsmittel(n) mit höherem beziehungsweise höheren Siedepunkt(en) als der beziehungsweise die des beziehungsweise der ersteren bei Temperaturen nicht über 300°C und bei Verwendung des Wasserstoffakzeptors beziehungsweise der Wasserstoffakzeptoren in Mengen von 0,35 bis 2 Mol je Mol des 10,11--Di-(hydro)-5H-dibenz[b,f]azepines der Formel II durchgeführt wird.

Die hochsiedenden polaren protischen Lösungsmittel haben 2 vorteilhafte Wirkungen auf die katalytische Dehydrierungsreaktion. Einerseits fördern sie die an der Oberfläche des Katalysators beziehungsweise der Katalysatoren ablaufenden Adsorptions-/Desorptions-Vorgänge, an denen das 10,11-Di-(hydro)-5H-dibenz[b,f]azepin, das 5H-Dibenz[b,f]azepin, der beziehungsweise die Wasserstoffakzeptor(en) und dessen beziehungsweise deren reduzierte Produkte teilnehmen. Da im erfindungsgemäßen Verfahren die Desorption des 5H-Dibenz[b,f]azepines und des Wasserstoffakzeptors beziehungsweise der Wasserstoffakzeptoren leichter abläuft als gemäß der EP-OS 237 952, kann die Menge des Wasserstoffakzeptors beziehungsweise der Wasserstoffakzeptoren gemäß den obigen Festlegungen verringert sein, wodurch die Vergiftung des Katalysators ausgeschaltet oder unterdrückt wird, was zu einer Beschleunigung der Reaktion und einer Erhöhung der Umsetzung führt. Andererseits ist die Konzentration der Ausgangssubstanz im Reaktionsgemisch viel höher, da die Umsetzung mit wesentlich weniger Wasserstoffakzeptor(en) und Lösungsmittel(n) oder überhaupt ohne Lösungsmittel vorgenommen wird. Deshalb verläuft die Reaktion innerhalb 1 bis 2 Stunde(n), eine Zersetzung des Produktes findet kaum statt und das Produkt kann in guter Ausbeute und hoher Reinheit isoliert werden.

Vorteilhaft wird beziehungsweise werden als Wasserstoffakzeptor(en) 1 oder mehr hochsiedende Nitroverbindung(en) und/oder hochsiedende ungesättigte Verbindung(en) verwendet. Als hochsiedende Nitroverbindung(en) ist beziehungsweise sind [eine] aromatische, insbesondere [ein] Nitrotoluol(e), wie 2-(Nitro)-toluol, Nitrobenzol(e), wie Nitrobenzol und/oder 1,3-Di-(nitro)-benzol, und Nitro xylol(e) und als

hochsiedende ungesättigte Verbindung(en) Benzalaceton

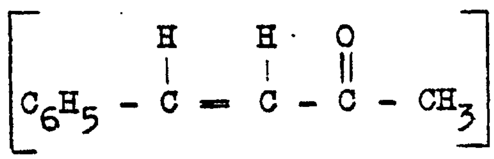 ,

Benzylidenacetophenon

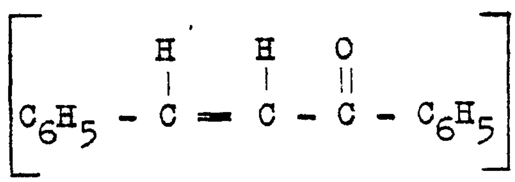

und/oder Stilben bevorzugt.

Zweckmäßig wird beziehungsweise werden als hochsiedende[s] polare[s] protische[s] Lösungsmittel [ein] solche[s] mit [einem] Siedepunkt(en) von mindestens 180°C eingesetzt.

Es ist auch vorteilhaft, als polare[s] protische[s] Lösungsmittel Äthylenglykol und/oder [ein] Oligo- und/oder Polyäthylenglykol(e), letztere[s] mit Molekulargewichten von 200 bis 1 000, und/oder [ein] Derivat(e) desselben beziehungsweise derselben zu verwenden. Als Oligoäthylenglykol(e) ist beziehungsweise sind Diäthylenglykol und/oder Triäthylenglykol bevorzugt. Als Polyäthylenglykol(e) ist beziehungsweise sind [ein] solche(s) mit Molekulargewichten von 200 bis 600 bevorzugt. Als Derivat(e) von Äthylenglykol und/oder [einem] Oligo- und/oder Polyäthylenglykol(en) ist beziehungsweise sind Äther, insbesondere Diäthylenglykolmonomethyläther, Triäthylenglykolmonomethyläther und [ein] Polyäthylenglykolmethyläther, letztere[s] vor allem mit Molekulargewichten von 350 bis 750, bevorzugt.

Die Reaktionsdauer kann weiter verkürzt werden, wenn das beziehungsweise die hochsiedende(n) polare(n) protische(n) Lösungsmittel in Mischung mit dem beziehungsweise den apolaren Lösungsmittel(n) mit höherem beziehungsweise höheren Siedepunkt(en) als dar beziehungsweise die des beziehungsweise der ersteren verwendet wird beziehungsweise werden. Vorteilhaft wird beziehungsweise werden als apolare[s] Lösungsmittel ein Gemisch aus 27 Gew.-% Diphenyl und 73 Gew.-% Diphenyloxyd [Diphyl] und/oder [ein] Polychlorbenzol(e) verwendet.

Ein weiterer Vorteil der Verwendung des beziehungsweise der hochsiedenden polaren protischen Lösungsmittel[s] besteht darin, daß die gesamte Menge des 5H-Dibenz[b,f]azepinproduktes durch Zugabe von Wasser nach dem Ende der Reaktion aus dem Reaktionsgemisch abgetrennt werden kann. Nach einer zweckmäßigen Ausführungsform des erfindungsgemäßen Verfahrens wird daher das Reaktionsgemisch in der Weise aufgearbeitet, daß, gegebenenfalls nach Zugabe von 1 oder mehr Lösungsmittel(n), der beziehungsweise die Edelmetallkatalysator(en) abfiltriert und das 5H-Dibenz[b,f]azepinprodukt kristallisiert wird oder das 5H-Dibenz[b,f]azepinprodukt durch Zugabe von Wasser oder 1 oder mehr organischen Lösungsmittel(n), in welchem beziehungsweise welchen es schlecht löslich ist, gefällt wird.

Durch die Verwendung des beziehungsweise der hochsiedenden protischen Lösungsmittel[s] und der geringeren Menge Wasserstoffakzeptor(en) kann die Vergiftung des Katalysators so weit zurückgedrängt werden, daß der Katalysator sogar mehrmals wiederverwendet werden kann. Nach einer zweckmäßigen Ausführungsform des erfindungsgemäßen Verfah rens wird daher der beziehungsweise die für das Dehydrieren eingesetzte[n] Edelmetallkatalysator(en), gegebenenfalls mehrere Male, wiederverwendet.

Für die Umsetzung des erfindungsgemäßen Verfahrens werden zweckmäßig Temperaturen von 180 bis 300°C vorzugsweise 200 bis 270°C, angewendet.

Nach einer Ausführungsform des erfindungsgemäßen Verfahrens wird eine Schmelze von 10,11-Di-(hydro)-5H-dibenz[b,f]azepin bei 150 bis 300°C, vorzugsweise 200 bis 250°C, in Gegenwart eines Edelmetallkatalysators (Palladium oder Platin, gegebenenfalls auf einem Träger) und von 0,35 bis 2 Mol Wasserstoffakzeptor je Mol 10,11-Di-(hydro)-5H-dibenz[b,f]azepin 1 bis 2 Stunde(n) lang umgesetzt. Nach dem Ende der Reaktion wird das Reaktionsgemisch heiß in einem polaren organischen Lösungsmittel, zum Beispiel Aceton, Chloroform, Dimethylsulfoxyd, Dimethylformamid und/oder 1 oder mehr Alkohol(en), gelöst, der Katalysator abfiltriert und das Produkt kristallisiert oder durch ein Lösungsmittel, in dem es sich schlecht löst, zum Beispiel 1 oder mehr niedere Alkohol(e), Kohlenwasserstoff(e) und/oder Wasser, gefällt.

Nach einer anderen Ausführungsform des erfindungsgemäßen Verfahrens wird das 10,11-Di-(hydro)-5H-dibenz[b,f]azepin in einem hochsiedenden polaren protischen Lösungsmittel in Gegenwart eines Edelmetallkatalysators und in von 0,35 bis 2 Mol Wasserstoffakzeptor je Mol 10,11-Di-(hydro)-5H-dibenz[b,f]azepin bei Temperaturen von 180 bis 300°C, vorzugsweise 200 bis 280°C, 1 bis 2 Stundenlang umgesetzt. Nach dem Ende der Reaktion wird der Katalysator abfiltriert. Aus dem Filtrat wird das Produkt entweder durch ein Lösungsmittel, in dem es sich schlecht löst, vorzugsweise Wasser, gefällt, oder aus dem Filtrat wird das Lösungsmittel abdestilliert und mit dem Rückstand auf die bereits beschriebene Weise verfahren.

Nach einer weiteren Ausführungsform des erfindungsgemäßan Verfahrens wird die Umsetzung das 10,11-Di-(hydro)-5H-dibenz[b,f]azepines in einem Gemisch aus einem hochsiedenden polaren protischen Lösungsmittel und einem noch höher siedenden apolaren Lösungsmittel unter den Reaktionsbedingungen der vorstehend beschriebenen Ausführungsform durchgeführt. Die Isolierung des Produktes kann nach einer der oben beschriebenen Verfahrensweisen vorgenommen werden.

Die Erfindung wird an Hand der folgenden Beispiele näher erläutert.

## Beispiel 1

Es wurde ein Gemisch aus 60 g (0,308 Mol) 10,11-Di-(hydro)-5H-dibenz[b,f]azepin, 30 ml (0,254 Mol) 2-(Nitro)-toluol und 3,6 g 10 gew.-%-iger Palladiumaktivkohle auf 100°C erwärmt. Nach Einschalten des Rührwerkes wurde die Schmelze 1 bis 2 Stunde(n) lang unter Rückfluß auf 200 bis 220°C gehalten. Dann wurde die Schmelze in 400 ml Diäthylenglykol gelöst, der Katalysator wurde aus der heißen Lösung abfiltriert und das Filtrat wurde mit 250 ml Methanol versetzt und dann über Nacht bei 0 bis 5°C stehengelassen. Das ausgefallene Produkt wurde abfiltriert, mit wenig Methanol gewaschen und dann getrocknet. Ausbeute: 46,5 g (71,3% der Theorie), Schmelzpunkt: 194 bis 196°C.

## Beispiel 2

Es wurden 6 g (0,03 Mol) 10,11-Di-(hydro)-5H-dibenz[b,f]azepin, 3 ml (0,025 Mol) 2-(Nitro)-toluol und 0,2 g Platinschwarz bei 200 bis 220°C 1 bis 2 Stunde(n) lang zum Sieden erhitzt. Dann wurde die Schmelze abgekühlt, heiß in 100 ml Aceton gelöst, der Katalysator durch Filtrieren entfernt und das Filtrat eingeengt. Nach dem Abkühlen wurde das Produkt abfiltriert und getrocknet. Ausbeute: 5,05 g (85,0% der Theorie), Schmelzpunkt: 190 bis 192°C.

## Beispiel 3

Es wurde ein Gemisch aus 30 g (0,154 Mol) 10,11-Di-(hydro)-5H-dibenz[b,f]azepin, 200 ml Diäthylenglykol und 20 g (0,146 Mol) 2-(Nitro)-toluol mit 1,8 g 10 gew.-%-iger Palladiumaktivkohle versetzt und dann 1,5 Stunden lang bei 220 bis 240°C unter Rückfluß zum Sieden erhitzt. Der Katalysator wurde aus der heißen Lösung abfiltriert und das 5H-Dibenz[b,f]azepin wurde durch Zugabe von 200 ml Wasser gefällt, abfiltriert, mit Wasser gewaschen und dann getrocknet. Ausbeute: 28,1 g (94,6% der Theorie), Schmelzpunkt: 194 bis 196°C.

## Beispiel 4

Es wurde ein Gemisch aus 30 g (0,154 Mol) 10,11-Di-(hydro)-5H-dibenz[b,f]azepin, 150 ml Triäthylenglykol und 10 g (0,06 Mol) 1,3-Di-(nitro)-benzol mit 1,9 g 10 gew.-%-iger Palladiumaktivkohle versetzt und dann 1 Stunde lang bei 240 bis 270°C gerührt beziehungsweise geschüt telt. Dann wurde der Katalysator aus der heißen Lösung abfiltriert und das Filtrat mit 100 ml Methanol versetzt und abgekühlt. Das ausgefallene 5H-Dibenz[b,f]azepin wurde abfiltriert, mit 30 ml Methanol gewaschen und dann getrocknet. Ausbeute: 26,3 g (88,6% dar Theorie), Schmelzpunkt: 198 bis 199,5°C.

Beispiel 5

Es wurden 12 g (0,06 Mol) 10,11-Di-(hydro)-5H-dibenz[b,f]azepin und 7,4 g (0,06 Mol) Nitrobenzol in Gegenwart von 0,75 g 10 gew.-%-iger Palladiumaktivkohle in einem Gemisch aus 30 ml eines Gemisches aus 27 Gew.-% Diphenyl und 73 Gew.-% Diphenyloxyd [Diphyl] und 30 ml Diäthylenglykol 1 Stunde lang unter Rückfluß zum Sieden erhitzt. Der Katalysator wurde heiß abfiltriert und die abgekühle Lösung mit 60 ml Methanol versetzt. Das Produkt wurde abfiltriert, mit 20 ml Methanol gewaschen und dann getrocknet. Ausbeute: 9,94 (85,0% der Theorie), Schmelzpunkt: 196 bis 198°C.

Beispiel 6

Es wurde ein Gemisch aus 19,5 g (0,1 Mol) 10,11-Di-(hydro)-5H-dibenz[b,f]azepin, 1 g 10 gew.-%-iger Palladiumaktivkohle, 21,9 g (0,15 Mol) Benzalaceton, 50 ml Diäthylenglykol 50 ml eines Gemisches aus 27 Gew.% Diphenyl und 73 Gew.-% Diphenyloxyd [Diphyl] unter Rückfluß und Rühren beziehungsweise Schütteln 2 Stunden lang zum Sieden erhitzt. Der Katalysator wurde heiß abfiltriert und das Filtrat mit 100 ml Methanol versetzt. Nach dem Abkühlen wurde das Produkt abfiltriert, mit Methanol gewaschen und dann getrocknet. Ausbaute: 14,51 g (75,2% der Theorie), Schmelzpunkt: 196 bis 198°C.

Beispiel 7

Es wurden 12,0 g (0,06 Mol) 10,11-Di-(hydro)-5H-dibenz[b,f]azepin und 8,2 g (0,06 Mol) 2-(Nitro)-toluol [o-Nitrotoluol] in Gegenwart von 1,0 g 10 gew.-%-igem Palladiumaktivkohle-Katalysator in 50 ml Triäthylenglykolmonomethyläther 1 Stunde lang bei 250°C gerührt beziehungsweise geschüttelt. Der Katalysator wurde heiß abfiltriert und zum Filtrat wurden 50 ml Methanol zugegeben und das aus der abgekühlten Lösung gefällte Produkt wurde abfiltriert, mit Methanol gewaschen und getrocknet. Ausbeute: 9,0 g (77% der Theorie), Schmelzpunkt: 196 bis 198°C.

Beispiel 8

Es wurden 19,5 g (0,1 Mol) 10,11-Di-(hydro)-5K-dibenz[b,f]azepin, 1 g 10 gew.-%-iger Palladiumaktivkohle-Katalysator und 27 g (0,15 Mol) trans-Stilben in einem Gemisch aus 50 ml Diäthylenglykol und 50 ml eines Gemisches aus 27 Gew.-% Diphenyl und 73 Gew.-% Diphenyloxyd [Diphyl) 2 Stunden lang unter Rückfluß gerührt beziehungsweise geschüttelt. Der Katalysator wurde heiß abfiltriert und dem Filtrat wurden 100 ml Methanol zugesetzt Nach dem Abkühlen der Lösung wurde das abgesonderte Produkt abfiltriert, mit Methanol gewaschen und getrocknet. Ausbeute: 15,8 g (80% der Theorie), Schmelzpunkt: 196 bis 197°C.

Beispiel 9

Es wurden 19,5 g (0,1 Mol) 10,11-Di-(hydro)-5H-dibenz[b,f]azepin, 1 g 10 gew.-%-iger Palladiumaktivkohle-Katalysator und 31,2 g (0,15 Mol) Benzylidenacetophenon in 120 ml Polyäthylenglykolmethyläther (durchschnittliches Molekulargewicht: 350) 3 Stunden lang bei 250°C gerührt beziehungsweise geschüttelt. Der Katalysator wurde heiß abfiltriert und dem Filtrat wurden 120 ml Methanol zugesetzt. Nach dem Abkühlen wurde das ausgeschiedene Produkt abfiltriert und mit Methanol gewaschen. Ausbeute: 14,8 g (75% der Theorie), Schmelzpunkt: 196 bis 197°C.

**Ansprüche**

1.) Verfahren zur Herstellung von 5H-Dibenz[b,f]azepin der Formel

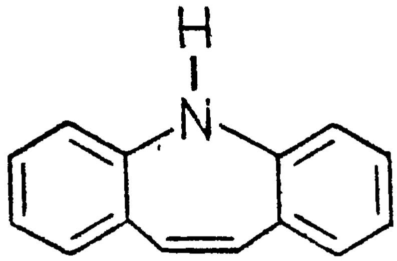

I

durch Dehydrieren von 10,11-Di-(hydro)-5H-dibenz[b,f]azepin der Formel

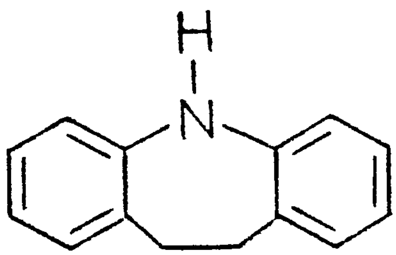

II

in Gegenwart von 1 oder mehr Edelmetallkatalysator(en) und Wasserstoffakzeptor(en) und gegebenenfalls Lösungsmittel(n), dadurch gekennzeichnet, daß man die Umsetzung in der Schmelze oder in 1 oder mehr hochsiedenden polaren protischen Lösungsmittel(n) oder einer Mischung aus 1 oder mehr hochsiedenden polaren protischen Lösungsmittel(n) und 1 oder mehr apolaren Lösungsmittel(n) mit höherem beziehungsweise höheren Siedepunkt(en) als der beziehungsweise die des beziehungsweise der ersteren bei Temperaturen nicht über 300°C und bei Verwendung des Wasserstoffakzeptors beziehungsweise der Wasserstoffakzeptoran in Mengen von 0,35 bis 2 Mol je Mol das 10,11-Di-(hydro)-5H-dibenz[b,f]azepines der Formel II durchführt.

2.) Verfahren nach Anspruch 1, dadurch gekennzeichnet, daß man als Wasserstoffakzeptor(en) 1 oder mehr hochsiedende Nitroverbindung(en) und/oder hochsiedende ungesättigte Verbindung(en) verwendet.

3.) Verfahren nach Anspruch 1 oder 2, dadurch gekennzeichnet, daß man als polare[s] protische[s] Lösungsmittel Äthylenglykol und/oder [ein] Oligo-und/oder Polyäthylenglykol(e), letztere[s] mit Molekulargewichten von 200 bis 1 000, und/oder [ein] Derivat(e) desselben beziehungsweise derselben verwendet.

4.) Verfahren nach Anspruch 1 bis 3, dadurch gekennzeichnet, daß man als apolare[s] Lösungsmittel ein Gemisch aus 27 Gew.-% Diphenyl und 73 Gew.-% Diphenyloxyd und/oder [ein] Polychlorbenzol(e) verwendet.

5.) Verfahren nach Anspruch 1 bis 4, dadurch gekennzeichnet, daß man das Reaktionsgemisch in der Weise aufarbeitet, daß man, gegebenenfalls nach Zugabe von 1 oder mehr Lösungsmittel(n), den beziehungsweise die Edelmetallkatalysator(en) abfiltriert und das 5H-Dibenz[b,f]azepinprodukt kristallisiert oder das 5H-Dibenz[b,f]azepinprodukt durch Zugabe von Wasser oder 1 oder mehr organischen Lösungsmittel(n), in welchem beziehungsweise welchen es schlecht löslich ist, fällt.

6.) Verfahren nach Anspruch 1 bis 5, dadurch gekennzeichnet, daß man den beziehungsweise die für das Dehydrieren eingesetzten Edelmetallkatalysator(en) wiederverwendet.

Europäisches Patentamt

# EUROPÄISCHER RECHERCHENBERICHT

Nummer der Anmeldung

EP 90 10 8372

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X<br>Y | EP-A-0 237 952 (ORION-YHTYMÄ OY FERMION)<br>* Insgesamt, und insbesondere Beispiel 4 *<br>--- | 1-6 | C 07 D 223/22 |
| D,Y | US-A-3 074 931 (P.N. CRAIG)<br>* Insgesamt, und insbesondere Spalte 3, Zeilen 3-16,32-38 *<br>----- | 1-6 | |
| | | | RECHERCHIERTE SACHGEBIETE (Int. Cl.5) |
| | | | C 07 D 223/00 |

Der vorliegende Recherchenbericht wurde für alle Patentansprüche erstellt

| Recherchenort | Abschlußdatum der Recherche | Prüfer |
|---|---|---|
| DEN HAAG | 01-08-1990 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)